# EUROPEAN PATENT SPECIFICATION

(11) **EP 2 878 264 B1**
(45) Date of publication and mention of the grant of the patent: **15.11.2017**
(21) Application number: 13195072.7
(22) Date of filing: 29.11.2013
(51) Int. Cl.: G03B 42/02, G03B 42/04, A61B 6/00

(54) **Mobile x-ray apparatus for storing an x-ray detector and method for bagging an x-ray detector into a hygiene protective cover**
Verfahrbare Röntgenvorrichtung zum Lagern eines Röntgendetektors und Verfahren zum Verpacken eines Röntgendetektors in einer hygienischen Schutzabdeckung
Appareil à rayons x mobile pour stocker un détecteur de rayons x et procédé d'ensachage pour détecteur de rayons x dans un couvercle de protection hygiénique

(43) Date of publication of application: 03.06.2015
(73) Proprietor: Agfa HealthCare N.V., 2640 Mortsel (BE)
(72) Inventor: Von Stein, Florian, 82194 Gröbenzell (DE); Senft, Daniel, 2840 Terhagen (Rumst) (BE)

(56) References cited:
- WO-A1-2013/074032
- US-A1- 2013 064 351
- Hardware Guide: "DRX-Revolution Mobile X-ray System Hardware Guide", , 29 February 2012 (2012-02-29), XP055115023, Retrieved from the Internet: URL:http://www.spectrumxray.com/sites/defa ult/files/pdfs/Carestream-DRX-Revolution.p df [retrieved on 2014-04-23]
- "DRX-Revolution Mobile X-ray System", , 14 November 2013 (2013-11-14), XP055115011, carestream.com Retrieved from the Internet: URL:https://www.carestream.com/brochure-dr x-revolution.pdf [retrieved on 2014-04-23]

## Description

The present invention relates to a mobile x-ray apparatus and an according receptacle for storing an x-ray detector as well as a method for bagging an x-ray detector into a hygiene protective cover according to the independent claims.

Mobile x-ray solutions play an important role in diagnostic imaging of patients who lack mobility. Efficient mobile bedside imaging solutions for improved patient comfort have been developed recently. For example, a mobile x-ray apparatus comprises a mobile unit on servo-driven wheels and a tube arm on which an x-ray generator is provided. In order to ensure enhanced flexibility, a wireless solid state x-ray detector is provided. In order to have an improved infection control, the x-ray detector can be covered with hygiene protection means, e.g. a sterile plastic bag of corresponding form and size, before an x-ray is recorded. Because of the relatively high weight of x-ray detectors of typically 3,5 kg and more, however, covering or bagging such detectors with plastic foils or bags is usually inconvenient for the medical staff.

US 2013/064351 A1 describes a mobile radiography apparatus including a movable transport frame that includes storage area including a plurality of individual portions or slots such as a detector storage holder or large detector and grid storage, a large detector storage and/or a small detector storage.

Hardware guide "DRX-Revolution Mobile X-ray System Hardware Guide", XP055115023, relates to a mobile x-ray system comprising shelves for holding a detector in place while bagging and unbagging the detector.

Brochure "DRX-Revolution Mobile X-ray System", XP055115011, discloses a mobile x-ray system which holds the detector for easy bagging.

WO 2013/074032 A1 relates to a mobile radiography apparatus comprising a detector slot for storage of detectors, which is designed to eject the detectors by means of a spring mechanism.

It is an object of the present invention to provide a mobile x-ray apparatus and an according receptacle for storing an x-ray detector as well as a method allowing for both safe storage and convenient bagging of the x-ray detector.

The object is achieved by a mobile x-ray apparatus, a receptacle and a method according to the independent claims.

The mobile x-ray apparatus according to the invention comprises an x-ray source and a receptacle for storing at least one x-ray detector, the receptacle being designed for holding an x-ray detector in a first position, in which a first portion of the x-ray detector is located inside of the receptacle and a second portion of the x-ray detector is located outside of the receptacle. According to a preferred aspect of the invention, the apparatus comprises a holding element for holding the x-ray detector in a second position, in which a third portion of the x-ray detector is located inside of the receptacle and in which a fourth portion of the x-ray detector is located outside of the receptacle, wherein the fourth portion is larger than the second portion.

The receptacle for storing at least one x-ray detector according to the invention is designed for holding an x-ray detector in a first position, in which a first portion of the x-ray detector is located inside of the receptacle and a second portion of the x-ray detector is located outside of the receptacle. According to a preferred aspect of the invention, the receptacle comprises a holding element for holding the x-ray detector in a second position, in which a third portion of the x-ray detector is located inside of the receptacle and a fourth portion of the x-ray detector is located outside of the receptacle, wherein the fourth portion is larger than the second portion.

The method for bagging an x-ray detector into a hygiene protective cover comprises storing an x-ray detector in a receptacle, in which the x-ray detector is held in a first position, in which a first portion of the x-ray detector is located inside of the receptacle and a second portion of the x-ray detector is located outside of the receptacle. According to a preferred aspect of the invention, the method comprises the following steps: positioning, in particular manually positioning, the x-ray detector into a second position, in which a third portion of the x-ray detector is located inside of the receptacle and a fourth portion of the x-ray detector is located outside of the receptacle, wherein the fourth portion is larger than the second portion; holding the x-ray detector in the second position by means of a holding element provided in the receptacle; and bagging, in particular manually bagging, the x-ray detector into a hygiene protective cover, in particular a plastic foil or bag, while the x-ray detector is held in the second position.

The x-ray apparatus does not necessarily have to be a mobile x-ray apparatus. Rather, the x-ray apparatus may be a stationary x-ray device, like a Bucky table or wall stand designed for receiving and/or holding an x-ray detector or a so-called CR digitizer for reading out x-ray information recorded in photostimulable phosphor sheets stored in cassettes.

Therefore, the invention also relates to an x-ray apparatus comprising a receptacle for storing at least one x-ray detector, the receptacle being designed for holding an x-ray detector in a first position, in which a first portion of the x-ray detector is located inside of the receptacle and a second portion of the x-ray detector is located outside of the receptacle. According to a preferred aspect of the invention, the apparatus comprises a holding element for holding the x-ray detector in a second position, in which a third portion of the x-ray detector is located inside of the receptacle and in which a fourth portion of the x-ray detector is located outside of the receptacle, wherein the fourth portion is larger than the second portion.

The term "x-ray detector" in the sense of the present invention preferably relates to a detector which is designed for converting x-ray radiation passing an object, e.g. a patient, and impinging on the detector into corresponding electric signals. X-ray detectors of this type are also referred to as "direct semiconductor detectors" and are preferably designed for contactless transmission of the electric signals generated by the x-ray detector to a remote processing unit, e.g. a computer workstation. Preferably, the x-ray detector is a high-resolution detector comprising a plurality of detector pixels made of a-Si having a pixel size of approximately 139 µm.

Alternatively, the term "x-ray detector" in the sense of the present invention may also relate to a cassette accommodating an x-ray sensitive medium, e.g. a photographic film or a photostimulable phosphor sheet, in which x-ray radiation passing an object, e.g. a patient, and impinging on the medium can be recorded. After radiographic exposure, the medium has to undergo a film processing or an optical read-out step, respectively, in order to visualize the recorded x-ray information.

Moreover, the term "x-ray detector" in the sense of the present invention may also relate to an x-ray detector assembly which comprises the x-ray detector itself and a carrier for the x-ray detector. For example, the carrier may comprise a holding frame surrounding the x-ray detector and/or a base plate on which the x-ray detector is mounted. Preferably, the carrier is provided with a handle allowing for a convenient and safe manual handling of the x-ray detector assembly, in particular in order to shift it from the first position into the second position. For example, an x-ray detector has a height of approx. 15 mm, whereas an x-ray detector assembly comprising the x-ray detector and a surrounding holding frame provided on a base plate may have a height of between 20 and 30 mm, preferably of approx. 26 mm.

The invention is based on the approach to store an x-ray detector in a receptacle, which is provided at an, in particular mobile, x-ray apparatus, in a first position, in which a sufficiently large, in particular a predominant, portion of the x-ray detector is located within the receptacle such that the x-ray detector is reliably held in the receptacle, in particular in order to ensure a safe transport of the x-ray detector when the mobile x-ray apparatus is moved. Moreover, the receptacle is designed such that the x-ray detector stored therein can be manually pulled up, inclined and, by means of a holding element, locked in a second position in which a predominant portion of the x-ray detector protrudes out of the receptacle such that the x-ray detector is more readily accessible from the outside than in the first position. In particular, in the second position the x-ray detector is readily accessible for being bagged by means of a hygiene protective cover, e.g. a plastic bag. Once the x-ray detector has been appropriately bagged, it can be easily manually removed from the receptacle and is available for a subsequent x-ray recording.

In summary, the invention allows for a safe storage as well as a convenient bagging of x-ray detectors.

Preferably, the receptacle being designed for holding the x-ray detector in the first position such that the first portion of the x-ray detector located inside of the receptacle is larger than the second portion of the x-ray detector located outside of the receptacle. By this means a very safe storage of the x-ray detector in the first position is achieved.

It is, moreover, preferred that the receptacle and/or the holding element are designed for holding the x-ray detector in the second position such that the third portion of the x-ray detector located inside of the receptacle is smaller than the fourth portion of the x-ray detector located outside of the receptacle. In other words, in the second position of the x-ray detector, the fourth portion being outside of the receptacle is larger than the third portion being inside of the receptacle ensuring that a predominant portion of the x-ray detector is accessible from outside of the receptacle allowing for a particularly comfortable bagging of the x-ray detector.

According to another preferred embodiment, the receptacle is provided with a slot for receiving and/or holding the x-ray detector in the first position. In particular, the holding element is provided near or adjacent to the slot. By this means, a safe storage of the x-ray detector is achieved in a particularly simple manner.

Preferably, the x-ray detector has the shape of a flat rectangular prism with two opposing main faces, i.e. a back face and an opposing front face, as well as four narrow side faces extending along the edges of the main faces.

According to an aspect of the invention, the receptacle further comprises at least one positioning element on which a back face and/or a side face of the x-ray detector abuts when the detector is in the first position. In this way, the reliability of the storage of the x-ray cassette is further enhanced without considerably increasing complexity.

According to yet another preferred embodiment the holding element is designed for locking the x-ray detector in the second position. By this means, the x-ray detector can be held in the second position in a particularly reliable way rendering the bagging procedure both convenient and safe.

Preferably, the holding element comprises a first contact face, on which a front face of the x-ray detector can abut and/or rest when the detector is in the second position. In particular, by means of contacting the front face of the x-ray detector, the first contact face holds and/or locks the x-ray detector in a pre-defined inclined orientation. This embodiment also contributes to ensure a convenient bagging of x-ray detectors.

Alternatively or additionally, the holding element comprises a second contact face on which a side face, in particular a lower side face, of the x-ray detector abuts and/or rests when the x-ray detector is in its second position. In particular, by means of contacting the, preferably lower, side face of the x-ray detector, the second contact face holds and/or locks the x-ray detector such that a pre-defined portion, i.e. the fourth portion, of the x-ray detector protrudes out of the receptacle. By means of at least one of these embodiments, a particularly safe and convenient bagging of the protruding portion of the x-ray detector is allowed.

Preferably, the holding element comprises a recess with which a, in particular bottommost, portion of the x-ray detector is in mesh when the x-ray detector is in the second position. This allows for a particularly reliable holding and/or locking of the x-ray detector in the second position which renders the bagging procedure both safe and particularly convenient.

It is, moreover, preferred that the holding element is designed for holding the x-ray detector in the second position such that the orientation of the x-ray detector in the second position is different from the orientation of the x-ray detector in the first position. Preferably, the x-ray detector exhibits an inclination against the vertical direction, wherein the inclination against the vertical direction is larger in the second position of the x-ray detector than in the first position of the x-ray detector. By this means, a particularly easy access to the protruding x-ray detector portion is achieved allowing for a convenient bagging of the x-ray detector.

Further advantages, aspects and examples of the present invention will be apparent from the description of following figures:
- Fig. 1: shows an example of a mobile x-ray apparatus in a perspective view;
- Fig. 2: shows a side view of an x-ray apparatus with a receptacle storing an x-ray detector in a first position;
- Fig. 3: shows a side view of an x-ray apparatus with a receptacle storing an x-ray detector in a second position;
- Fig. 4: shows a perspective view of an x-ray apparatus with a receptacle stor-ing an x-ray detector in a first position;
- Fig. 5: shows a perspective view of an x-ray apparatus with a receptacle stor-ing an x-ray detector in a second position;
- Fig. 6: shows a side view of an x-ray apparatus with a receptacle storing an-other x-ray detector in a first position; and
- Fig. 7: shows a side view of an x-ray apparatus with a receptacle storing an-other x-ray detector in a second position.

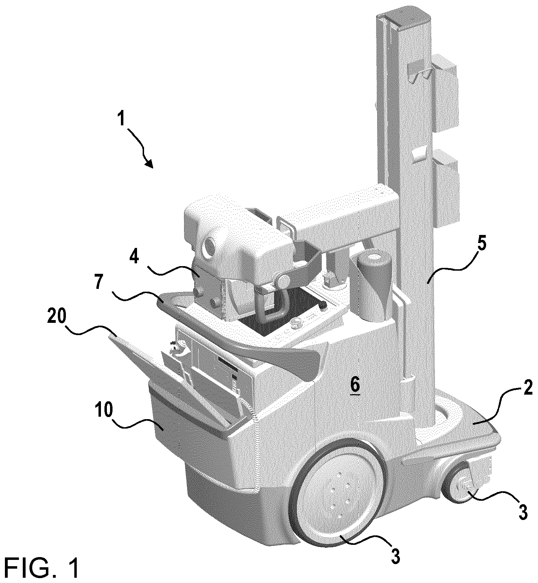

Figure 1 shows an example of a mobile x-ray apparatus 1 in a perspective view. The apparatus 1 comprises a mobile platform 2 on four wheels 3, wherein preferably two of the four wheels 3 are driven by a motor, e.g. a servo motor. The apparatus 1 further comprises an x-ray generator 4, also referred to as x-ray source, which is pivotably and/or vertically adjustably mounted on a tube arm 5 provided on the platform 2. The mobile x-ray apparatus 1 further comprises a body 6 on which a handle 7 is provided by means of which the apparatus 1 can be pushed and/or steered by an operator.

On a side area of the body 6 of the apparatus 1 a receptacle 10 is provided. As shown in Figure 1, the receptacle 10 is preferably provided at the back side of the body 6 below handle 7.

The receptacle 10 is designed for storing and/or holding an x-ray detector 20, for example a direct semiconductor detector, preferably an a-Si detector, which converts impinging x-ray radiation into electrical signals and is preferably designed for a wireless transfer of the electric signals to a remote processing unit, for example a computer workstation.

Preferably, size and shape of the x-ray detector 20 correspond to size and shape of conventional x-ray cassettes storing an x-ray sensitive medium, for example an x-ray film or a photostimulable phosphor sheet. Preferably, the x-ray detector 20 has a size of 46 x 38.4 x 1.5 cm with an effective imaging region of approximately 42 x 35 cm.

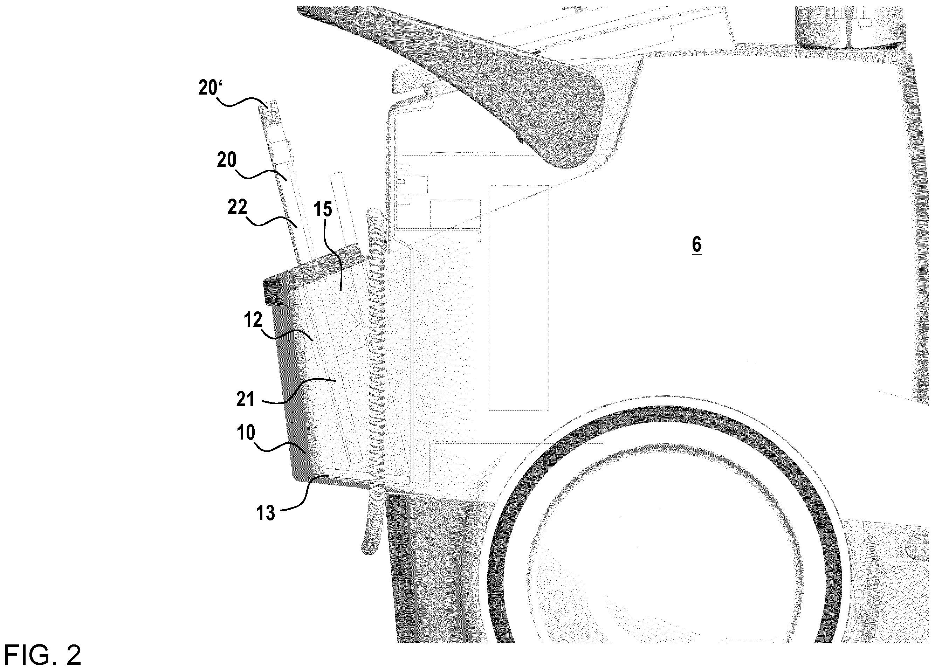

Structure and functionality of the receptacle 10 will be elucidated in more detail in the following with reference to Figures 2 to 5.

Figure 2 shows a schematic side view of a portion of an x-ray apparatus in the region of body 6 on the backside of which a receptacle 10 is provided. For sake of clarity, the receptacle 10 is displayed transparently such that objects or elements provided within the receptacle 10 are visible.

As apparent from Figure 2, an x-ray detector 20 is stored in the receptacle 10 in a first position, wherein a first portion 21 of the x-ray detector 20 is located within the receptacle 10, whereas a second portion 22 of the x-ray detector 20 is located outside of the receptacle 10.

In the present example, the receptacle 10 holds the x-ray detector 20 in the first position by means of a first positioning element 12 provided at an upper section of the receptacle 10 and a second positioning element 13 provided at a lower section, in particular the bottom, of the receptacle 10, such that a back face of the x-ray detector 20 is supported by the first positioning element 12 and a lower side face of the x-ray detector 20 is supported by the second positioning element 13.

Preferably, the first positioning element 12 constitutes a first lateral part of a storing slot the lower end of which is constituted by the second positioning element 13. Preferably, an opposite lateral part of the storing slot is given by holding element 15.

As apparent from Figure 2, the first portion 21 of the x-ray detector 20 located within the receptacle 10 is larger than the second portion 22 of the x-ray detector 20 located outside of the receptacle 10. Accordingly, in the first position a reliable storage of the x-ray detector 20 in the receptacle 10 is ensured, in particular when the mobile x-ray apparatus is set in motion.

Preferably, the receptacle 10, in particular the first positioning element 12 and/or the second positioning element 13, is designed for holding the x-ray detector 20 in the first position such that the x-ray detector 20 is inclined against the vertical direction by an angle between 10 to 20 degrees, in particular by approx. 15 degrees.

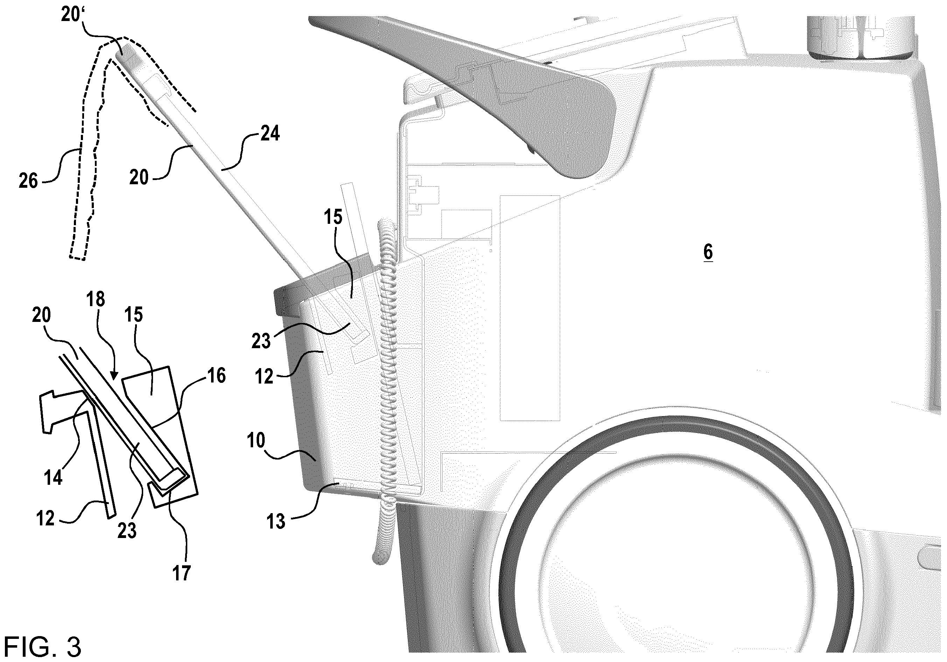

Figure 3 shows a side view of the x-ray apparatus shown in Figure 2, wherein the receptacle 10 holds the x-ray detector 20 in a second position, in which a third portion 23 of the x-ray detector 20 is located inside of the receptacle 10, whereas a fourth portion 24, which is larger than the third portion 23, is located outside of the receptacle 10. Moreover, the x-ray detector 20 is inclined against the vertical direction by an angle between 30 to 50 degrees, in particular by approx. 40 degrees. Preferably, the angle of inclination of the x-ray detector 20 in the second position is larger, in particular by approx. 25 to 35 degrees, than the angle of inclination of the x-ray detector 20 in the first position.

The receptacle 10 is designed such that the x-ray detector 20 stored in the first position (see Figure 2) can be manually placed into the shown second position in which it is held and/or locked. For this purpose, the holding element 15 is designed for receiving a lower portion and/or a lower end face of the x-ray detector 20 after it has been manually pulled up and brought into an increased inclined orientation. Moreover, at the upper end of the receptacle 10 a slanted face 14 is provided, preferably at the first positioning element 12, on which the back face of the inclined x-ray detector 20 can abut.

This is illustrated in more detail by means of the inset of Figure 3 showing an enlarged representation of the holding element 15. As apparent from the inset, the holding element 15 comprises a slanted first contact face 16 and a second contact face 17 which runs in essential perpendicularly to the first contact face 16. Preferably, the second contact face 17 constitutes the base of a recess provided in the holding element 15 in which the x-ray detector 20 can be inserted and held and/or locked in its second position. Preferably, the recess in the holding element 15 has a depth of between 2 to 10 mm, in particular between approximately 3 and 5 mm. The inset of Figure 3 further shows the first positioning element 12 which, together with the holding element 15, forms a storing slot 18 in which the x-ray detector 20 can be inserted and held in the first position, as well as the slanted face 14, which is preferably provided adjacently to the first positioning element 12.

In summary, the x-ray detector 20 stored in the receptacle 10 in a first position can be manually pulled up, slightly inclined and, by means of the holding element 15, locked in a second position in which a forth portion 24, which is larger than a second portion 22 (see Figure 2), of the x-ray detector 20 protrudes out of the receptacle 10 in a manner in which the x-ray detector 20 is more readily accessible from the outside, in particular for being bagged by means of a plastic bag 26 for hygiene protection purposes. Once the x-ray detector 20 has been appropriately bagged, it can be easily removed from the receptacle 10 together with the plastic bag 26 and is available for subsequent x-ray recordings.

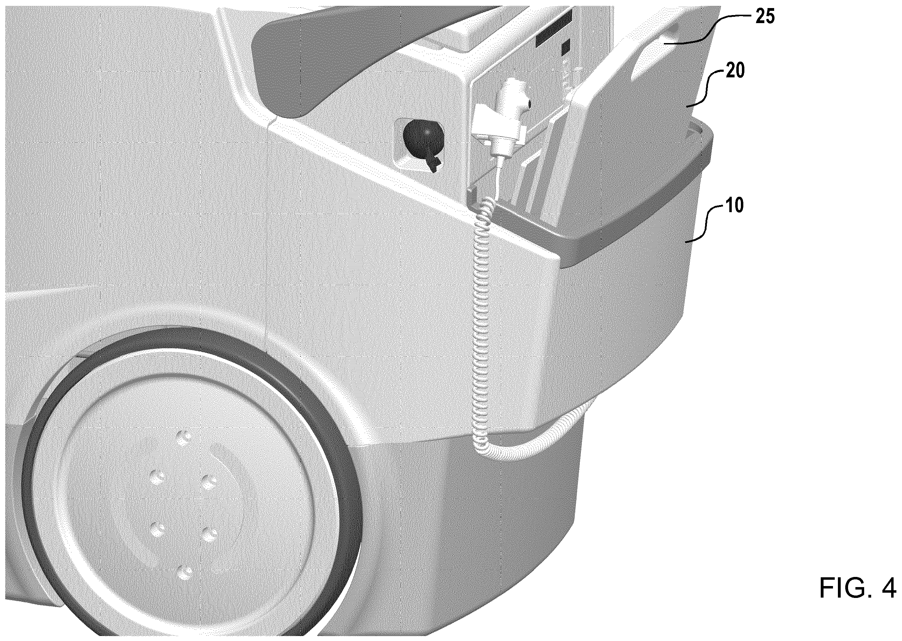

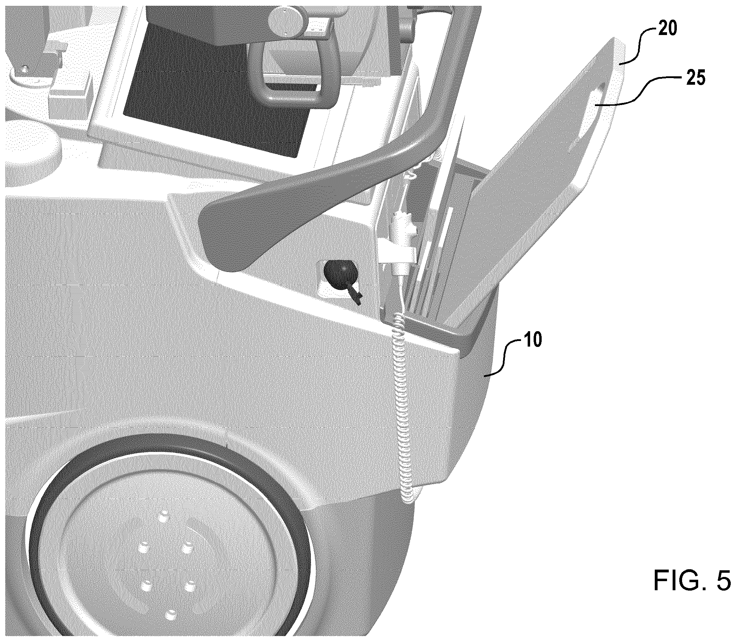

Figure 4 shows a perspective view of a detail of an x-ray apparatus comprising a receptacle 10 holding an x-ray detector 20 in a first position in which the x-ray detector 20 is in a first position where only a small portion of the x-ray detector 20 is accessible from outside of the receptacle 10. In distinction to this, Figure 5 shows a perspective view of the receptacle 10 holding the x-ray detector 20 in a second position, in which a considerably larger portion of the x-ray detector 20 protrudes out of the receptacle 10, which is therefore readily accessible for being bagged with a plastic bag or other hygiene protection means.

As apparent from Figures 4 and 5, the x-ray detector 20 may be provided with a handle 25 by means of which it can be readily taken by hand in order to shift it from the first position shown in Figures 2 and 4 into the second position shown in Figures 3 and 5.

Preferably, the handle 25 is provided on a carrier 20' (see Figures 2 and 3), e.g. a base plate with a frame-like holding structure provided on the base plate, for carrying the x-ray detector 20. Accordingly, in the embodiments shown in Figures 2 to 5, the x-ray detector 20 in the sense of the invention is given by an x-ray detector assembly comprising the x-ray detector 20 and the carrier 20'.

Moreover, the elucidations with reference to Figures 2 to 5 given above apply accordingly to an x-ray detector 20 in the narrower sense, i.e. an x-ray detector 20 without additional carrier means.

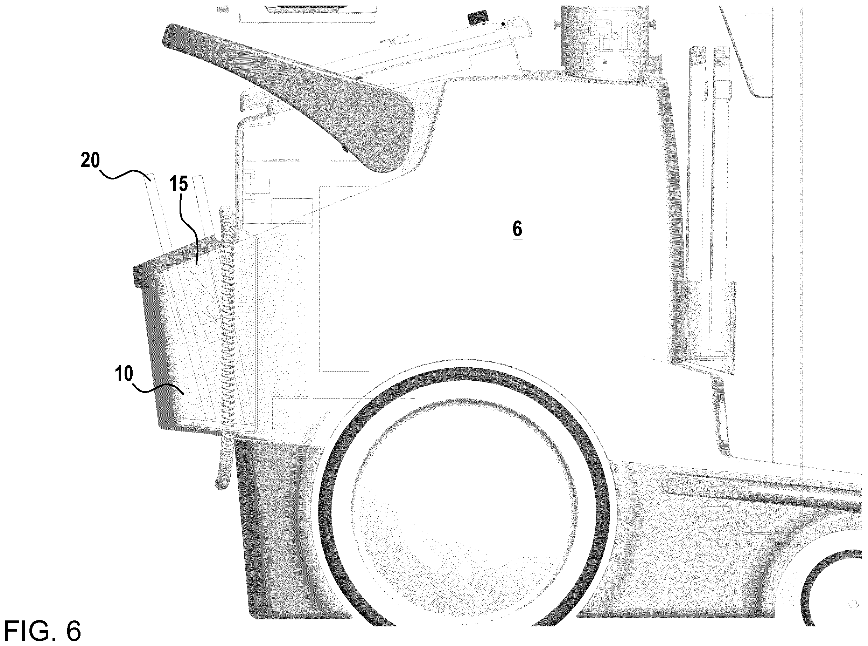

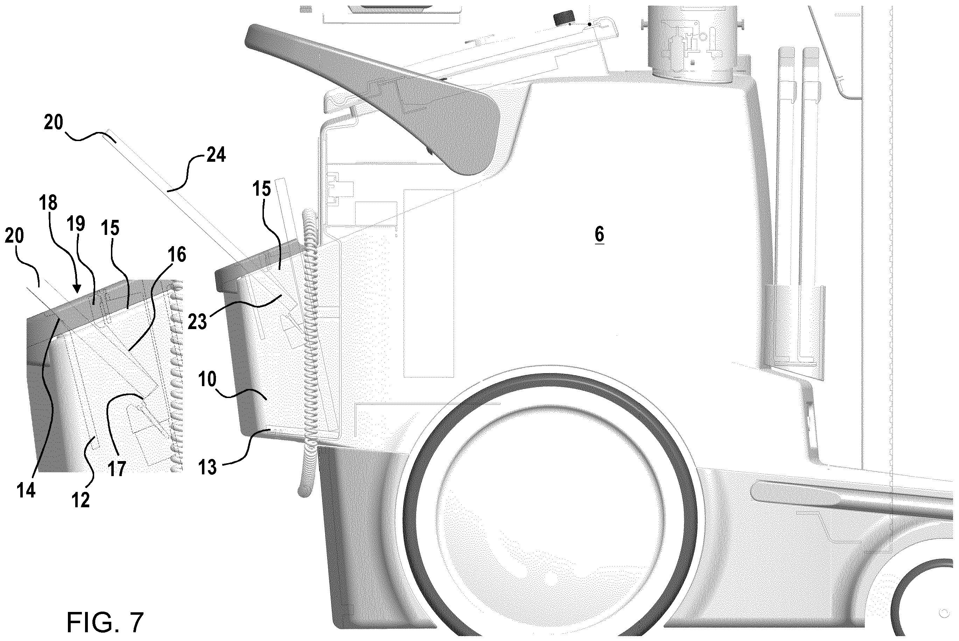

This is exemplarily shown in Figures 6 and 7, where an x-ray detector 20, preferably having a size and shape of a conventional x-ray cassette, is stored in the receptacle 10 in a first and second position, respectively. Basically, the elucidations set forth above with reference to Figures 2 to 5 apply accordingly.

According to an aspect of the invention, the holding element 15 is provided with a detachable adapter element 19 for adapting the size and/or shape of the holding element 15 to the size and/or shape of the x-ray detector 20. In the given examples, the x-ray detector 20 shown in Figures 6 and 7 is different from the x-ray detector assembly 20 shown in Figures 2 to 5 regarding size and shape.

As apparent from the inset of Figure 7 showing an enlarged section of an upper part of the receptacle 10, the adapter element 19 is provided at an upper end of the first contact face 16 of the holding element 15 and is designed such that the space, i.e. the width of storing slot 18, between the first positioning element 12 and the holding element 15 is reduced in order to account for the reduced height of the x-ray detector 20 compared to the one shown in Figures 2 to 5. Additionally or alternatively, it is also possible to provide a corresponding adapter element (not shown) at the lower end of the holding element 15, in particular at the second contact face 17. By means of according adapter elements it can be ensured that various kinds of x-ray detectors 20, i.e. with or without carrier and/or handle, are reliably stored and/or held in the receptacle in the first and/or second position.

Preferably, the receptacle 10 according to the invention is designed for receiving and/or storing and/or holding x-ray detectors 20 and/or x-ray detector assemblies of different formats. In particular, the dimensions of the receptacle 10 and/or the holding slot 18 and/or the first and/or second positioning element 12 or 13 and/or the holding element 15 and/or the first and/or second contact face 16 or 17 and/or the adapter element 19 are chosen such that an x-ray detector 20 can be stored and/or held in the receptacle 10 both in vertical and in horizontal format.

## Claims

1. A mobile x-ray apparatus (1) comprising an x-ray source (4) and a receptacle (10) for storing at least one x-ray detector (20), the receptacle (10) being designed for holding an x-ray detector (20) in a first position, in which a first portion (21) of the x-ray detector (20) is located inside of the receptacle (10) and a second portion (22) of the x-ray detector (20) is located outside of the receptacle (10), and the receptacle (10) comprising a holding element (15) for holding the x-ray detector (20) in a second position, in which a third portion (23) of the x-ray detector (20) is located inside of the receptacle (10) and a fourth portion (24) of the x-ray detector (20) is located outside of the receptacle (10), wherein the fourth portion (24) is larger than the second portion (22), and a first positioning element (12) on which a back face of the x-ray detector (20) abuts when the x-ray detector (20) is in the first position,
**characterized by**
an adapter element (19), which is detachably provided at the holding element (15) and designed to reduce the space between the first positioning element (12) and the holding element (15) to account for a reduced height of an x-ray detector (20).

2. The mobile x-ray apparatus according to claim 1, the receptacle (10) being designed for holding the x-ray detector (20) in the first position such that the first portion (21) of the x-ray detector (20) located inside of the receptacle (10) is larger than the second portion (21) of the x-ray detector (20) located outside of the receptacle (10).

3. The mobile x-ray apparatus according to claim 1 or 2, the receptacle (10) and/or the holding element (15) being designed for holding the x-ray detector (20) in the second position such that the third portion (23) of the x-ray detector (20) located inside of the receptacle (10) is smaller than the fourth portion (24) of the x-ray detector (20) located outside of the receptacle (10).

4. The mobile x-ray apparatus according to any of the preceding claims, further comprising a slot (18) for holding and/or receiving the x-ray detector (20) in the first position.

5. The mobile x-ray apparatus according to claim 4, wherein the holding element (15) is provided adjacent to or near to the slot (18).

6. The mobile x-ray apparatus according to any of the preceding claims, wherein the holding element (15) is designed for locking the x-ray detector (20) in the second position.

7. The mobile x-ray apparatus according to any of the preceding claims, wherein the holding element (15) comprises a first contact face (16) on which a front face of the x-ray detector (20) abuts when the x-ray detector (20) is in the second position.

8. The mobile x-ray apparatus according to any of the preceding claims, wherein the holding element (15) comprises a second contact face (17) on which a side face of the x-ray detector (20) abuts when the x-ray detector (20) is in the second position.

9. The mobile x-ray apparatus according to any of the preceding claims, wherein the holding element (15) comprises a recess with which a portion of the x-ray detector (20), in particular a bottommost portion of the x-ray detector (20), is in mesh when the x-ray detector (20) is in the second position.

10. The mobile x-ray apparatus according to any of the preceding claims, wherein the holding element (15) is designed for holding the x-ray detector (20) in the second position such that the orientation of the x-ray detector (20) in the second position is different from the orientation of the x-ray detector (20) in the first position.

11. The mobile x-ray apparatus according to claim 7 and/or 8, wherein the adapter element (19) is provided at an upper end of the first contact face (16) of the holding element (15) or a lower end of the holding element (15) at the second contact face (17), or alternatively the adapter element (19) is provided at the upper end and a corresponding adapter element is provided at the lower end.

12. A receptacle (10) for storing at least one x-ray detector (20), the receptacle (10) being designed for holding an x-ray detector (20) in a first position, in which a first portion (21) of the x-ray detector (20) is located inside of the receptacle (10) and a second portion (22) of the x-ray detector (20) is located outside of the receptacle (10), and the receptacle (10) comprising a holding element (15) for holding the x-ray detector (20) in a second position, in which a third portion (23) of the x-ray detector (20) is located inside of the receptacle (10) and a fourth portion (24) of the x-ray detector (20) is located outside of the receptacle (10), wherein the fourth portion (24) is larger than the second portion (22), and a first positioning element (12) on which a back face of the x-ray detector (20) abuts when the x-ray detector (20) is in the first position,
**characterized by**
an adapter element (19), which is detachably provided at the holding element (15) and designed to reduce the space between the first positioning element (12) and the holding element (15) to account for a reduced height of an x-ray detector (20).

13. A method for bagging an x-ray detector (20) into a hygiene protective cover (26), comprising:
- storing an x-ray detector (20) in a receptacle (10), in which the x-ray detector (20) is held in a first position, in which a first portion (21) of the x-ray detector (20) is located inside of the receptacle (10), a second portion (22) of the x-ray detector (20) is located outside of the receptacle (10) and a back face of the x-ray detector (20) abuts on a first positioning element (12),
- positioning, in particular manually positioning, the x-ray detector (20) into a second position, in which a third portion (23) of the x-ray detector (20) is located inside of the receptacle (10) and a fourth portion (24) of the x-ray detector (20) is located outside of the receptacle (10), wherein the fourth portion (24) is larger than the second portion (22),
- holding the x-ray detector (20) in the second position by means of a holding element (15) provided in or at the receptacle (10), wherein an adapter element (19), detachably provided at the holding element (15), is designed to reduce the space between the first positioning element (12) and the holding element (15) to account for a reduced height of an x-ray detector (20), and
- bagging, in particular manually bagging, the x-ray detector (20) into a hygiene protective cover (26), in particular a plastic foil or bag, while the x-ray detector (20) is held in the second position.

## Patentansprüche

1. Eine verfahrbare Röntgenvorrichtung (1), umfassend eine Röntgenquelle (4) und eine Aufnahme (10) zum Lagern von mindestens einem Röntgendetektor (20), wobei die Aufnahme (10) derart ausgelegt ist, dass sie einen Röntgendetektor (20) in einer ersten Position, in der ein erster Bereich (21) des Röntgendetektors (20) innerhalb der Aufnahme (10) vorliegt und ein zweiter Bereich (22) des Röntgendetektors (20) außerhalb der Aufnahme (10) vorliegt, zurückhält und die Aufnahme (10) ein Halteglied (15) umfasst, mit dem der Röntgendetektor (20) in einer zweiten Position, in der ein dritter Bereich (23) des Röntgendetektors (20) innerhalb der Aufnahme (10) und ein vierter Bereich (24) des Röntgendetektors (20) außerhalb der Aufnahme (10) vorliegt, zurückgehalten wird, wobei der vierte Bereich (24) größer ist als der zweite Bereich (22), und
ein erstes Positionierelement (12), auf dem dann eine Rückfläche des Röntgendetektors (20) anliegt, wenn sich der Röntgendetektor (20) in der ersten Position befindet,
**gekennzeichnet durch**
ein Adapterelement (19), das lösbar am Halteglied (15) angeordnet ist und derart ausgelegt ist, dass es den Raum zwischen dem ersten Positionierelement (12) und dem Halteglied (15) verringert und auf diese Weise eine verringerte Höhe eines Röntgendetektors (20) ergibt.

2. Die verfahrbare Röntgenvorrichtung nach Anspruch 1, wobei die Aufnahme (10) so ausgelegt ist, dass sie den Röntgendetektor (20) derart in der ersten Position zurückhält, dass der erste Bereich (21) des Röntgendetektors (20) innerhalb der Aufnahme (10) größer ist als der zweite Bereich (21) des Röntgendetektors (20) außerhalb der Aufnahme (10).

3. Die verfahrbare Röntgenvorrichtung nach Anspruch 1 oder 2, wobei die Aufnahme (10) und/oder das Halteglied (15) derart ausgelegt ist (sind), dass sie (es) den Röntgendetektor (20) derart in der zweiten Position zurückhalt (zurückhalten), dass der dritter Bereich (23) des Röntgendetektors (20) innerhalb der Aufnahme (10) kleiner ist als der vierte Bereich (24) des Röntgendetektors (20) außerhalb der Aufnahme (10).

4. Die verfahrbare Röntgenvorrichtung nach einem der vorstehenden Ansprüche, die ferner einen Schlitz (18), in dem der Röntgendetektor (20) in der ersten Position zurückgehalten und/oder aufgenommen wird, umfasst.

5. Die verfahrbare Röntgenvorrichtung nach Anspruch 4, wobei das Halteglied (15) angrenzend an oder nahe am Schlitz (18) angeordnet ist.

6. Die verfahrbare Röntgenvorrichtung nach einem der vorstehenden Ansprüche, wobei das Halteglied (15) derart ausgelegt ist, dass es den Röntgendetektor (20) in der zweiten Position arretiert.

7. Die verfahrbare Röntgenvorrichtung nach einem der vorstehenden Ansprüche, wobei das Halteglied (15) eine erste Kontaktfläche (16) umfasst, auf der dann eine Vorderfläche des Röntgendetektors (20) anliegt, wenn sich der Röntgendetektor (20) in der zweiten Position befindet.

8. Die verfahrbare Röntgenvorrichtung nach einem der vorstehenden Ansprüche, wobei das Halteglied (15) eine zweite Kontaktfläche (17) umfasst, auf der dann eine Seitenfläche des Röntgendetektors (20) anliegt, wenn sich der Röntgendetektor (20) in der zweiten Position befindet.

9. Die verfahrbare Röntgenvorrichtung nach einem der vorstehenden Ansprüche, wobei das Halteglied (15) eine Aussparung umfasst, in die dann ein Bereich des Röntgendetektors (20), insbesondere ein Bodenbereich des Röntgendetektors (20), passt, wenn sich der Röntgendetektor (20) in der zweiten Position befindet.

10. Die verfahrbare Röntgenvorrichtung nach einem der vorstehenden Ansprüche, wobei das Halteglied (15) derart ausgelegt ist, dass es den Röntgendetektor (20) derart in der zweiten Position zurückhält, dass sich die Orientierung des Röntgendetektors (20) in der zweiten Position von der Orientierung des Röntgendetektors (20) in der ersten Position unterscheidet.

11. Die verfahrbare Röntgenvorrichtung nach Anspruch 7 und/oder 8, wobei das Adapterelement (19) an einem oberen Ende der ersten Kontaktfläche (16) des Halteglieds (15) oder an einem unteren Ende des Halteglieds (15) an der zweiten Kontaktfläche (17) angeordnet ist oder wobei alternativ das Adapterelement (19) am oberen Ende und ein entsprechendes Adapterelement am unteren Ende angeordnet ist.

12. Eine Aufnahme (10) zum Lagern von mindestens einem Röntgendetektor (20), wobei die Aufnahme (10) derart ausgelegt ist, dass sie einen Röntgendetektor (20) in einer ersten Position, in der ein erster Bereich (21) des Röntgendetektors (20) innerhalb der Aufnahme (10) vorliegt und ein zweiter Bereich (22) des Röntgendetektors (20) außerhalb der Aufnahme (10) vorliegt, zurückhält und die Aufnahme (10) ein Halteglied (15) umfasst, mit dem der Röntgendetektor (20) in einer zweiten Position, in der ein dritter Bereich (23) des Röntgendetektors (20) innerhalb der Aufnahme (10) und ein vierter Bereich (24) des Röntgendetektors (20) außerhalb der Aufnahme (10) vorliegt, zurückgehalten wird, wobei der vierte Bereich (24) größer ist als der zweite Bereich (22), und
ein erstes Positionierelement (12), auf dem dann eine Rückfläche des Röntgendetektors (20) anliegt, wenn sich der Röntgendetektor (20) in der ersten Position befindet,
**gekennzeichnet durch**
ein Adapterelement (19), das lösbar am Halteglied (15) angeordnet ist und derart ausgelegt ist, dass es den Raum zwischen dem ersten Positionierelement (12) und dem Halteglied (15) verringert und auf diese Weise eine verringerte Höhe eines Röntgendetektors (20) ergibt.

13. Ein Verfahren zum Verpacken eines Röntgendetektors (20) in eine hygienische Schutzabdeckung (26), umfassend:
- das Lagern eines Röntgendetektors (20) in einer Aufnahme (10), in der der Röntgendetektor (20) in einer ersten Position, in der ein erster Bereich (21) des Röntgendetektors (20) innerhalb der Aufnahme (10) vorliegt, ein zweiter Bereich (22) des Röntgendetektors (20) außerhalb der Aufnahme (10) vorliegt und eine Rückfläche des Röntgendetektors (20) auf einem ersten Positionierelement (12) anliegt, zurückgehalten wird,
- das Positionieren, insbesondere das manuelle Positionieren, des Röntgendetektors (20) in einer zweiten Position, in der ein dritter Bereich (23) des Röntgendetektors (20) innerhalb der Aufnahme (10) vorliegt und ein vierter Bereich (24) des Röntgendetektors (20) außerhalb der Aufnahme (10) vorliegt, wobei der vierte Bereich (24) größer ist als der zweite Bereich (22),
- das Zurückhalten des Röntgendetektors (20) in der zweiten Position mittels eines in oder an der Aufnahme (10) angeordneten Halteglieds (15), wobei ein lösbar am Halteglied (15) angeordnetes Adapterelement (19) derart ausgelegt ist, dass es den Raum zwischen dem ersten Positionierelement (12) und dem Halteglied (15) verringert und auf diese Weise ein verringerte Höhe eines Röntgendetektors (20) ergibt, und
- das Verpacken, insbesondere das manuelle Verpacken, des Röntgendetektors (20) in eine hygienische Schutzabdeckung (26), insbesondere in eine Kunststofffolie oder einen Kunststoffbeutel, während der Röntgendetektor (20) in der zweiten Position zurückgehalten wird.

## Revendications

1. Appareil à rayons X mobile (1) comprenant une source de rayons X (4) et un réceptacle (10) servant à stocker au moins un détecteur de rayons X (20), ledit réceptacle (10) étant conçu de façon à retenir un détecteur de rayons X (20) dans une première position dans laquelle une première portion (21) du détecteur de rayons X (20) se situe à l'intérieur du réceptacle (10) et une deuxième portion (22) du détecteur de rayons X (20) se situe à l'extérieur du réceptacle (10), et ledit réceptacle (10) comprenant un élément de retenue (15) servant à retenir le détecteur de rayons X (20) dans une deuxième position dans laquelle une troisième portion (23) du détecteur de rayons X (20) se situe à l'intérieur du réceptacle (10) et une quatrième portion (24) du détecteur de rayons X (20) se situe à l'extérieur du réceptacle (10), ladite quatrième portion (24) étant plus grande que la deuxième portion (22), et
un premier élément de positionnement (12) soutenant une face arrière du détecteur de rayons X (20) lorsque le détecteur de rayons X (20) se situe dans la première position,
**caractérisé par**
un élément adaptateur (19) disposé de façon amovible à l'élément de retenue (15) et conçu de façon à réduire l'espace entre le premier élément de positionnement (12) et l'élément de retenue (15) afin d'assurer ainsi une hauteur réduite d'un détecteur de rayons X (20).

2. Appareil à rayons X mobile selon la revendication 1, ledit réceptacle (10) étant conçu de façon à retenir le détecteur de rayons X (20) dans la première position de façon à ce que la première portion (21) du détecteur de rayons X (20) située à l'intérieur du réceptacle (10) soit plus grande que la deuxième portion (21) du détecteur de rayons X (20) située l'extérieur du réceptacle (10).

3. Appareil à rayons X mobile selon la revendication 1 ou 2, ledit réceptacle (10) et/ou l'élément de retenue (15) étant conçu(s) de façon à retenir le détecteur de rayons X (20) dans la deuxième position de façon à ce que la troisième portion (23) du détecteur de rayons X (20) située à l'intérieur du réceptacle (10) soit plus petite que la quatrième portion (24) du détecteur de rayons X (20) située à l'extérieur du réceptacle (10).

4. Appareil à rayons X mobile selon l'une quelconque des revendications précédentes, comprenant en outre une fente (18) servant à retenir et/ou recevoir le détecteur de rayons X (20) dans la première position.

5. Appareil à rayons X mobile selon la revendication 4, **caractérisé en ce que** l'élément de retenue (15) est disposé de façon adjacente à la fente (18) ou proche de la fente (18).

6. Appareil à rayons X mobile selon l'une quelconque des revendications précédentes, **caractérisé en ce que** l'élément de retenue (15) est conçu de façon à verrouiller le détecteur de rayons X (20) dans la deuxième position.

7. Appareil à rayons X mobile selon l'une quelconque des revendications précédentes, **caractérisé en ce que** l'élément de retenue (15) comprend une première face de contact (16) soutenant une face avant du détecteur de rayons X (20) lorsque le détecteur de rayons X (20) se situe dans la deuxième position.

8. Appareil à rayons X mobile selon l'une quelconque des revendications précédentes, **caractérisé en ce que** l'élément de retenue (15) comprend une deuxième face de contact (17) soutenant une face latérale du détecteur de rayons X (20) lorsque le détecteur de rayons X (20) se situe dans la deuxième position.

9. Appareil à rayons X mobile selon l'une quelconque des revendications précédentes, **caractérisé en ce que** l'élément de retenue (15) comprend un creux dans lequel s'engage une portion du détecteur de rayons X (20), en particulier une portion inférieure du détecteur de rayons X (20), lorsque le détecteur de rayons X (20) se situe dans la deuxième position.

10. Appareil à rayons X mobile selon l'une quelconque des revendications précédentes, **caractérisé en ce que** l'élément de retenue (15) est conçu de façon à retenir le détecteur de rayons X (20) dans la deuxième position de façon à ce que l'orientation du détecteur de rayons X (20) dans la deuxième position diffère de l'orientation du détecteur de rayons X (20) dans la première position.

11. Appareil à rayons X mobile selon la revendication 7 et/ou 8, **caractérisé en ce que** l'élément adaptateur (19) est disposé à une extrémité supérieure de la première face de contact (16) de l'élément de retenue (15) ou à une extrémité inférieure de l'élément de retenue (15) à la deuxième face de contact (17), une solution alternative consistant à disposer l'élément adaptateur (19) à l'extrémité supérieure et à disposer un élément adaptateur correspondant à l'extrémité inférieure.

12. Réceptacle (10) servant à stocker au moins un détecteur de rayons X (20), ledit réceptacle (10) étant conçu de façon à retenir un détecteur de rayons X (20) dans une première position dans laquelle une première portion (21) du détecteur de rayons X (20) se situe à l'intérieur du réceptacle (10) et une deuxième portion (22) du détecteur de rayons X (20) se situe à l'extérieur du réceptacle (10), et ledit réceptacle (10) comprenant un élément de retenue (15) servant à retenir le détecteur de rayons X (20) dans une deuxième position dans laquelle une troisième portion (23) du détecteur de rayons X (20) se situe à l'intérieur du réceptacle (10) et une quatrième portion (24) du détecteur de rayons X (20) se situe à l'extérieur du réceptacle (10), ladite quatrième portion (24) étant plus large que la deuxième portion (22), et
un premier élément de positionnement (12) soutenant une face arrière du détecteur de rayons X (20) lorsque le détecteur de rayons X (20) se situe dans la première position,
**caractérisé par**
un élément adaptateur (19) disposé de façon amovible à l'élément de retenue (15) et conçu de façon à réduire l'espace entre le premier élément de positionnement (12) et l'élément de retenue (15) afin d'assurer ainsi une hauteur réduite d'un détecteur de rayons X (20).

13. Procédé d'ensachage d'un détecteur de rayons X (20) dans un couvercle de protection hygiénique (26), ledit procédé consistant à :
- stocker un détecteur de rayons X (20) dans un réceptacle (10) dans lequel le détecteur de rayons X (20) est retenu dans une première position dans laquelle une première portion (21) du détecteur de rayons X (20) se situe à l'intérieur du réceptacle (10), une deuxième portion (22) du détecteur de rayons X (20) se situe à l'extérieur du réceptacle (10) et une face arrière du détecteur de rayons X (20) est soutenue par un premier élément de positionnement (12),
- positionner, en particulier positionner manuellement, le détecteur de rayons X (20) dans une deuxième position dans laquelle une troisième portion (23) du détecteur de rayons X (20) se situe à l'intérieur du réceptacle (10) et une quatrième portion (24) du détecteur de rayons X (20) se situe à l'extérieur du réceptacle (10), ladite quatrième portion (24) étant plus grande que la deuxième portion (22),
- retenir le détecteur de rayons X (20) dans la deuxième position au moyen d'un élément de retenue (15) disposé dans ou au réceptacle (10), un élément adaptateur (19), disposé de façon amovible à l'élément de retenue (15), étant conçu de façon à réduire l'espace entre le premier élément de positionnement (12) et l'élément de retenue (15) afin d'obtenir ainsi une hauteur réduite d'un détecteur de rayons X (20), et
- ensacher, en particulier ensacher manuellement, le détecteur de rayons X (20) dans un couvercle de protection hygiénique (26), en particulier une feuille ou sac en plastique, pendant que le détecteur de rayons X (20) est retenu dans la deuxième position.
